# EUROPEAN PATENT APPLICATION

(11) **EP 4 603 059 A1**
(43) Date of publication of application: **20.08.2025**
(21) Application number: 23875928.6
(22) Date of filing: 11.10.2023
(51) Int. Cl.: A61F 2/24

(54) **TRANSCATHETER DEVICE**

(30) Priority: 12.10.2022 US 202263415556 P; 09.12.2022 US 202263431639 P
(71) Applicant: Tau Medical Inc., Busan, 46285 (KR); Tau Medical Inc., Centreville, Virginia 20120 (US)
(72) Inventor: KIM, June-Hong, Busan 48516 (KR)
(74) Representative: FRKelly
(86) International application number: PCT/KR2023/015626
(87) International publication number: WO 2024/080742

(57) **Abstract**

A transcatheter device includes: a main shaft including a proximal portion, an intermediate portion, and a distal portion, and having a first lumen for insertion of a guidewire, a second lumen for insertion of a nitinol wire, and a third lumen for insertion of an injection tube formed therein; a distal tail comprising a distal portion of the main shaft; a spacer body mounted on the intermediate portion positioned between the distal portion and the proximal portion of the main shaft, in which the proximal portion of the main shaft includes a proximal segment and an intravascular anchor connected to the proximal segment, and wherein the spacer body comprises: a mesh structured body configured contractible or expandable and to be expanded to have a predetermined configuration in the absence of an external force from an environment; and a balloon configured to cover the mesh structured body to maintain an enclosed state, and to be controlled in size depending on an amount of a saline solution injected into an internal space thereof.

## Description

### CROSS REFERENCE TO RELATED APPLICATION

The present application claims priority to American Patent Application No. 63/431,639 filed Sep. 12, 2022. and No. 63/415,556 filed Dec. 10, 2022., the entire contents of which is incorporated herein for all purposes by this reference.

### BACKGROUND OF THE INVENTION

### Field of the Invention

The present invention relates to a transcatheter device for treating tricuspid valve regurgitation.

### Description of the Related Art

Heart valve regurgitation (leakage through a heart valve) occurs when a heart valve fails to close properly. One example is tricuspid valve regurgitation, which is typically caused by changes in the geometric configurations of the right ventricle, papillary muscles, and tricuspid valve annulus. These geometric alterations result in incomplete leaflet coaptation during ventricular systole, thereby producing regurgitation. In the past, repairing heart valves required open-heart surgery with cardiopulmonary bypass. In recent years, a variety of catheter-based techniques for valve repair are being introduced. These catheter-based procedures do not require opening the chest or the use of cardiopulmonary bypass.

### SUMMARY OF THE INVENTION

The present invention is directed to providing a more improved transcatheter device for treating tricuspid regurgitation.

**Transcatheter Device:** In one aspect, this invention is a transcatheter device comprising a main shaft, a proximal portion, a distal tail, and a spacer body mounted on the main shaft and located between the proximal portion and the distal tail. This transcatheter device could be used for treating tricuspid valve regurgitation in a patient's heart. All or part of the transcatheter device is supported by the main shaft. The spacer body is mounted on the shaft, which travels through the spacer body. The proximal portion of the transcatheter device encompasses a proximal segment of the main shaft. This could be expressed alternatively as the proximal segment of the main shaft comprising the proximal portion of the transcatheter device. The distal tail of the transcatheter device encompasses a distal segment of the main shaft. This could be expressed alternatively as the distal segment of the main shaft comprising the distal tail of 1:he transcatheter device.

The main shaft comprises a lumen and opening(s) for admitting a guidewire therethrough. There could be an opening for the lumen at the distal tip of the main shaft (in the distal tail part). There could also be a proximal opening located at the proximal portion of the transcatheter device. In embodiments where the transcatheter device comprises an intravascular anchor, this proximal opening for the lumen could be located between the spacer body and the intravascular anchor. For example, the opening could be located at the proximal end of the main shaft where it joins the intravascular anchor.

The total length of the main shaft could be in the range of 50-175 cm long. The main shaft may be constructed in any suitable way. For example, it could be made of a metal wire core (e.g., stainless steel or nitinol alloy), which is then covered a polymer material. For example, the metal wire core could be covered with thermoplastic polyurethane braiding or polytetrafluoroethylene (PTFE) coating. The metal wire core could extend through the full length of the main shaft. However, in some embodiments, the metal wire core terminates before reaching the tip of the distal tail (or distal tip of main shaft). For example, the metal wire core could terminate at a location that is within 0.5-4 cm of the distal tip.

**Distal Tail:** The distal tail may have any suitable length to provide sufficient anchoring within the pulmonary artery. In some embodiments, the length of the distal tail is 10-40 cm long; and in some cases, 15-30 cm long. The distal tail could have a pigtail or rounded tip to blunt the tip and reduce trauma as it travels into pulmonary artery. In some embodiments, the distal tail has one or more bends. The bend(s) could have an inner angle in the range of 80- 140°. The bends(s) could be located at any suitable location on the distal tail. In some embodiments, there is a bend located at a distance of 0.25-3.5 cm from the spacer body.

The distal tail may have a non-constant diameter over its length. In some embodiments, the distal tail comprises a proximal segment and a distal segment. The proximal segment may encompass 10-60% of the total length of the distal tail. The distal segment could have a thinner diameter than the proximal segment. There may be various reasons for this difference, such as the proximal segment having more or thicker sheathing or covering than the distal segment. The distal segment could be more flexible than the proximal segment of the distal tail. In some embodiments, the distal tail does not comprise any coil, loop, or stent.

The distal tail could be designed to have a streamlined shape. In some embodiments, the distal tail is a thin elongated cylinder shape (with or without a lumen) with no protruding features, such as hooks, wires, rings, ridges, etc. This may be useful in preventing thrombus formation or erosion of the distal tail into the wall of the pulmonary artery.

In some embodiments, the distal segment is more flexible than the proximal segment. In some cases, the proximal segment comprises a metal braiding, whereas the distal segment does not. The distal segment could comprise a polymer material that is softer than the proximal segment. The distal segment could have a smaller diameter than the proximal segment. In some cases, the length of the distal segment is shorter than the length of the proximal segment. The length of the distal segment could be 2-7 cm long. The length of the proximal segment could be 7-15 cm long. In some cases, the proximal segment constitutes 35 - 65% of the total length of the distal tail.

The proximal segments could have a different size than the distal segment. In some cases, the distal segment has a smaller diameter than the proximal segment. In some cases, the diameter of the distal segment is 45-85% of the diameter size of the proximal segment. For example, the distal segment could have a diameter of 2-5 mm, whereas the proximal segment could have a diameter of 3-6 mm.

In some cases, the distal tail further comprises a middle segment between the proximal segment and the distal segment. The middle segment is more flexible than the proximal segment but is stiffer than the distal segment. In some cases, the length of the middle segment is shorter than the length of the proximal segment. For example, the length of the middle segment could be 2-7 cm long.

**Spacer Body:** The spacer body is mounted on the main shaft. The spacer body is made to have dimensions or shape suitable for providing a coaptation surface for leaflets of the tricuspid valve. For example, the shape of the spacer body may have a particular design. In some embodiments, the spacer body has a linear shape (e.g., ovoid, cylindrical with tapered or conical ends, etc.). In some embodiments, the spacer body has a non-linear shape (e.g., curved or boot-shaped). In a non-linear shaped spacer body, the spacer body could comprise a bend having an inner angle in the range of 80-140°.

Another design parameter is the length of the spacer body. For example, the spacer body could be 4-13 cm long; and in some cases, 5-9 cm. In cases where the spacer body has a non-linear shape, this length is represented by the travel distance along the longitudinal axis of the spacer body. The width of the spacer body can be measured on a transverse cross-section plane that is orthogonal to the longitudinal axis. In some embodiments, the widest width of the spacer body on this transverse cross-section plane is in the range of 0.5-3.5 cm; and in some cases, in the range of 0.5-2.5 cm. The spacer body may have a relaxed contracted configuration and an elongated configuration. In this situation, the measurements above for the spacer body are made in the relaxed configuration. In some embodiments, the width of the spacer body on the widest axis is greater than the width of the spacer body on its cross-axis on the transverse plane (i.e., non-circular or asymmetric cross-section).

The spacer body can have any suitable structure, such as balloon (e.g., fluid, foam, or air-filled), basket, mesh, struts (e.g., like a stent), framework, skeleton, scaffolding, etc. If needed, a surface for the spacer body may be provided in any suitable manner, such as a skin, shell, casing, or membrane. The spacer body may be made of any suitable material, such as plastics, metals, or combinations thereof. The spacer body could have one or more openings to allow the flow of blood therethrough. There may be a gap between the spacer body (at one of its ends) and the main shaft to allow the flow of blood therethrough. These openings or gaps allows blood to flow easily through the spacer body, which may be useful for preventing thrombus formation.

In some embodiments, the spacer body comprises one or more side appendages. These may be located on a lateral side of the spacer body. The side appendage can be any type of thin and flexible structure that enhances the function of the spacer body as a barrier against the flow of blood across gaps in the tricuspid valve leaflets. Examples of side appendages include wings, flaps, shrouds, drapes, skirts, free edges, tags, etc. The side appendage has a widened configuration (for ventricular systole) and a narrowed configuration (for ventricular diastole).

The widened configuration for the side appendage is induced by the direction of blood flow and could be performed in any suitable manner such as spreading out, extending out, enlarging, distending, folding out, opening, etc. The narrowed configuration for the side appendage is induced by the other direction of blood flow and could be performed in any suitable manner such as folding in, retracting, collapsing, shrinking, closing, etc.

The side appendage should be sufficiently wide to reduce gaps between the tricuspid valve leaflets or help stabilize the spacer body across the tricuspid valve. In some embodiments, the width of the side appendage is 0.3 - 5.0 cm; and in some cases, 0.5 - 3.5 cm. The width is measured as the widest distance for the side appendage from spacer body in a direction that is orthogonal to the transverse axis of the spacer body.

The length of the side appendage may be shorter than the length of the spacer body. In some embodiments, the length of the side appendage is 2 - 9 cm; and in some cases, 4-7 cm. The length is the longest length as measured along the longitudinal axis of the spacer body.

The side appendage should be sufficiently thin to be flexibly responsive to blood flow across the tricuspid valve. In some embodiments, the side appendage has a thickness of 0.2 - 10 mm; and in some cases, 0.3 - 6 mm. The thickness is measured along a transverse axis of the spacer body that is orthogonal to the side appendage and the longitudinal axis of the spacer body.

The side appendage can have any suitable shape. In some embodiments, the side appendage has a non-flat shape with a three-dimensional curvature that gives the side appendage an inner side (concave) and an outer side (convex). Having this non-flat shape may be useful for improving the response to blood flow across the tricuspid valve.

**Proximal Portion:** The proximal portion of the transcatheter device comprises the proximal segment of the main shaft. The proximal segment could be a proximal continuation of the main shaft. The proximal portion of the transcatheter device could have any suitable length to provide intravascular access or sufficient anchoring within the vena cava. In some embodiments, the total length of the proximal portion is in the range of 10-60 cm long. In embodiments where the proximal portion includes an intravascular anchor, this measurement includes the length of the intravascular anchor. In situations where the intravascular anchor does not have a linear shape (e.g., coil), this means the length as measured along the longitudinal axis.

In some embodiments, the proximal segment of the main shaft has one or more bends. The bend(s) could have an inner angle in the range of 80-140°. The bends(s) could be located at any suitable location on the proximal segment of the main shaft. In some embodiments, there is a bend located at a distance of 0.25-5.5 cm from the spacer body. The proximal segment could also have a curved portion (wider than a bend). In some embodiments, the proximal segment has two separate bends and a curved portion between the two bends. The length of the proximal segment could be in the range of 3-15 cm long.

**Intravascular Anchor:** In some embodiments, the proximal portion comprises an intravascular anchor. Examples of intravascular anchors include spiral coil and expandable stent. In some embodiments, the intravascular anchor is a spiral coil. The spiral coil could have at least two spirals. The intravascular anchor could have any suitable width for anchoring in the vena cava. In some embodiments, the widest width of the intravascular anchor is in the range of 2-7 cm wide. The length of the intravascular anchor could be in the range of 4-11 cm long (as measured straight on its longitudinal axis). In situations where the intravascular anchor does not have a linear shape (e.g., coil), this means the length as measured along the longitudinal axis. In situations where the intravascular anchor has flexible configurations (e.g., as in a helical coil), this length is measured in its naturally coiled configuration. In an alternate embodiment of this invention, the transcatheter device comprises either the intravascular anchor or the distal tail, but not both.

**Radiopaque Markers:** The transcatheter device may have one or more radiopaque markers that are visible under x-ray imaging (e.g., x-ray fluoroscopy). In some embodiments of the transcatheter device, there is a first radiopaque marker that is located on the proximal segment of the main shaft (proximal to the spacer body), and a second radiopaque that is located on the distal tail (distal to the spacer body). The first radiopaque marker could be located within 2 cm of the proximal end of the spacer body. The second radiopaque marker could be located within 2 cm of the distal end of the spacer body.

**Coaptation Assembly:** In another aspect, this invention is a coaptation assembly for treating tricuspid valve regurgitation. The assembly comprises a transcatheter device of the invention. The assembly further comprises a guidewire traveling through the lumen of the main shaft. In some embodiments, the assembly further comprises a moveable delivery sheath that can cover the spacer body or intravascular anchor. The sheath could be advanced to cover the spacer body or intravascular anchor. Or the spacer body could be retracted to uncover the spacer body or intravascular anchor. In some embodiments, the assembly further comprises a deployment catheter. The deployment catheter is sufficiently long to deploy the transcatheter device in the patient's heart. For example, the deployment catheter could be 50-150 cm long.

**Coaptation Kit:** In another aspect, this invention is a coaptation kit for treating tricuspid valve regurgitation. The kit comprises a transcatheter device of the invention, a deployment catheter, a delivery sheath, and a guidewire. These components could be assembled or used in the manner described herein.

**Method of Treatment:** In another aspect, this invention is a method of treating a defective tricuspid valve in a patient using a transcatheter device of this invention. The transcatheter device is implanted with the distal tail within the pulmonary artery and the spacer body across the tricuspid valve. The transcatheter device is inserted into an entry vein, such as the femoral, subclavian, or jugular vein. The transcatheter device is advanced further into the vena cava (inferior or superior). The transcatheter device is advanced through a right atrium of the heart, across the tricuspid valve, and into a right ventricle of the heart. The transcatheter device is further advanced towards the pulmonary artery. The distal tail is advanced into the pulmonary artery. This could be the left-side or right-side pulmonary artery.

The distal tail works to help anchor the transcatheter device. As such, the distal tail may extend into the pulmonary artery of sufficient distance to perform this function. In some embodiments, the distal tail extends for a distance of at least 10 cm into the pulmonary artery; and in some cases, at least 15 cm. In some embodiments, the distal tail is advanced past a first branching point of the pulmonary artery; in some cases, past a second branching point of the pulmonary artery; and in some cases, past a third branching point of the pulmonary artery. Proper positioning of the distal tail could be confirmed by having a radiopaque marker and x-ray imaging to see the radiopaque marker. In some embodiments, the distal tail is not embedded within heart tissue.

The spacer body should be properly positioned between leaflets of the tricuspid valve. This proper placement could be confirmed by external imaging such as x-ray or echocardiogram. In some embodiments, the spacer body is positioned to abut against a supraventricular crest of the heart. This abutment against the supraventricular crest could occur at a location within the distal half of the spacer body. The tricuspid valve has a tricuspid annulus and there is an annular plane defined for the tricuspid annulus. This annular plane is along an x-axis of the tricuspid annulus and orthogonal to a Y-axis of the tricuspid annulus. In some embodiments, the spacer body is positioned at an oblique angle (<90°) relative to the annular plane. This oblique angle could be in the range of 15-75°.

In embodiments where the spacer body comprises a side appendage, the method could further comprise widening the side appendage during ventricular systole and narrowing the side appendage during ventricular diastole. In the widened configuration, the side appendage may be positioned between leaflets of the tricuspid valve and obstruct gaps that exist therein. In situations where the side appendage has a non-flat shape, the inner side (concave) is oriented to face towards the right ventricle.

In embodiments where the spacer body is provided with a balloon, a saline solution or air may be injected inside the spacer body to expand and maintain the balloon after the spacer body is properly positioned between the leaflets of the tricuspid valve. In this case, the amount of saline solution or air injected may be adjusted to allow the spacer body to have a predetermined size and volume to fit a size of the regurgitation space in the tricuspid valve.

In embodiments where the transcatheter device further comprises an intravascular anchor at its proximal portion, this intravascular anchor is lodged in the vena cava (inferior or superior). The transcatheter device could be implanted using a guidewire. The guidewire is inserted into an entry vein, such as the femoral vein and advanced further into the vena cava (inferior or superior). The guidewire is advanced through the right atrium of the heart, across the tricuspid valve, and into the right ventricle of the heart. The guidewire is further advanced towards the pulmonary artery. The guidewire is inserted into a guidewire lumen of the transcatheter device and the transcatheter device is advanced over this guidewire.

**Deployment:** The transcatheter device could be deployed using a delivery sheath and deployment catheter. During insertion, the delivery sheath could be moved to cover the spacer body, and for relevant embodiments, cover the intravascular anchor. During deployment the delivery sheath is retracted backwards. Retraction of the delivery sheath and unsheathing components of the transcatheter device could be part of the implantation process. In embodiments where the spacer body is self-expanding, this unsheathing could allow the spacer body to self-expand outward to provide a wider coaptation surface. In embodiments where the transcatheter device comprises an intravascular anchor having an expandable configuration, unsheathing allows the anchor to expand outward to lodge within the vena cava.

In some embodiments, this deployment assembly is not disassembled immediately after the procedure is completed. The clinician may wish to implement a short trial period to confirm the effectiveness of the device. For this short trial period, one or more components of the delivery assembly (deployment catheter, delivery sheath, or guidewire) could be retained inside the patient's body, along with the transcatheter device. During the short trial period, the tricuspid valve function is monitored (e.g., by echocardiogram). If the transcatheter device shows effectiveness during this trial period, the deployment assembly is removed, but retaining the transcatheter device in place. If the trial period shows ineffective results, having the deployment assembly still-in-place allows easy removal of the transcatheter device. The trial period could be any suitable short duration. For example, the trial period could be a duration that is within the range of 12-48 hours post-insertion.

**Retrieval:** After being implanted, the transcatheter device could be removed if needed. This can be done by grasping the intravascular anchor (e.g., spiral coil at its proximal tip) and pulling out the transcatheter device for removal from the patient's body. For example, this could be performed by inserting a snare catheter through an entry vein, advancing the snare catheter towards the spiral coil, grasping the spiral coil, withdrawing the snare catheter, and pulling out the transcatheter device from the entry vein.

In embodiments where the spacer body comprises a balloon, it is necessary to deflate the spacer body by drawing a saline solution or air out of the spacer body before performing the retrieval. In this case, a needle with a pointed tip wrapped in a delivery sheath can be approached to the outer side of the spacer body, and then the needle can be exposed from the delivery sheath to make a hole in the balloon of the spacer body and allow the saline solution to drain from the spacer body through the hole.

As described above, the present invention can be effectively applied to catheter-based treatment of tricuspid regurgitation.

### BRIEF DESCRIPTION OF THE DRAWINGS

FIG. 1A is a view illustrating an example of a transcatheter device for treating tricuspid valve regurgitation.
FIG. 1B is a view illustrating a main shaft and a distal tail of the transcatheter device in FIG. 1A.
FIG. 1C is a cross-sectional view taken along E-E' in FIG. 1A, illustrating a cross-sectional view of the main shaft of the transcatheter device.
FIG. 1D is a cross-sectional view of each portion of the distal tail in FIG. 1C, in which (a) is a cross sectional view taken along I-I', (b) is a cross-sectional view taken along II-II', and (c) is a cross-sectional view taken along III-III'.
FIG. 1E is a view illustrating another example of a transcatheter device for treating tricuspid valve regurgitation.
FIG. 1F is a view illustrating a main shaft and a distal tail of the transcatheter device in FIG. 1E.
FIG. 2Ais a view illustrating a spacer body having a balloon that covers a mesh structured body.
FIG. 2B is a view illustrating a spacer body with an e-PTEF layer that covers the balloon.
FIG. 2C is a cross-sectional view taken along A-A' in (b) of FIG. 2B.
FIG. 3A is a view illustrating an example of the transcatheter device in which an injection valve is mounted within the spacer body.
FIG. 3B is an enlarged view of a portion in which the injection valve in FIG. 3A is mounted.
FIG. 3C is a cross-sectional view taken along D-D' in FIG. 3B, illustrating open and closed states of the injection valve.
FIG. 3D is a cross-sectional view taken along B-B' in FIG. 3C, illustrating open and closed states of the injection valve.
FIG. 4A is a view illustrating an embodiment of the transcatheter device in which the injection valve is mounted on a proximal portion of the main shaft.
FIG. 4B is an enlarged view of a portion in which the injection valve in FIG. 4A is mounted, illustrating the open and closed states of the injection valve.
FIG. 5A is a view illustrating another embodiment of the transcatheter device in which the injection valve is mounted on the proximal portion of the main shaft.
FIG. 5B is an enlarged view of a portion in which the injection valve in FIG. 5A is mounted, illustrating the open and closed states of the injection valve.
FIG. 5C is a cross-sectional view taken along C-C' in FIG. 5B, illustrating open and closed states of the injection valve.
FIG. 6 is a view illustrating the transcatheter device disposed within the heart.

### DETAILED DESCRIPTION OF THE INVENTION

To assist in understanding the invention, reference is made to the accompanying drawings to show by way of illustration specific embodiments in which the invention may be practiced. The drawings herein are not necessarily made to scale or actual proportions. For example, lengths and widths of the components may be adjusted to accommodate the page size. Hereinafter, the terms 'distal' and 'proximal' are used to refer to a distant location as 'distal' and a closer location as 'proximal' with respect to an operator.

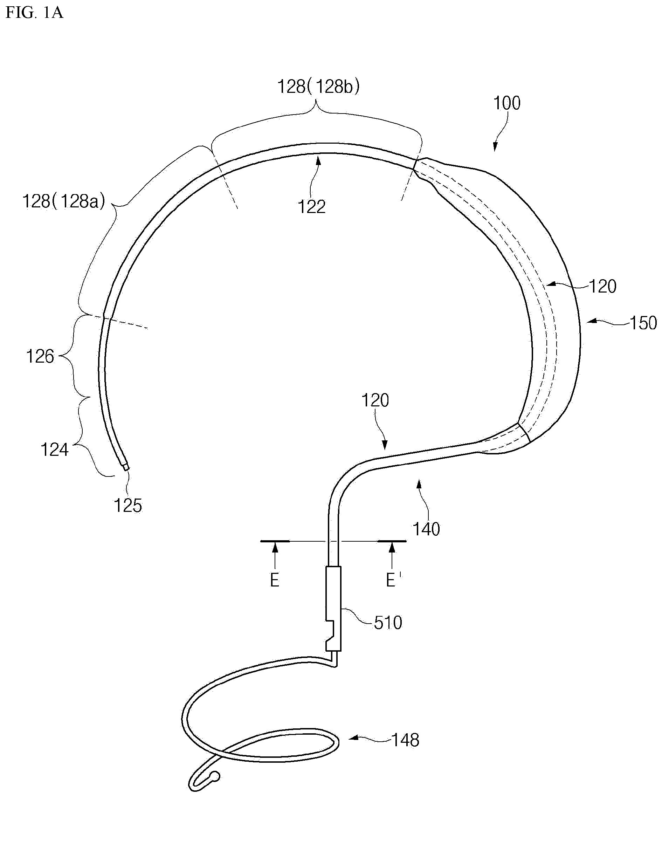

FIG. 1A is a view illustrating an embodiment of a transcatheter device 100. In this embodiment, the transcatheter device 100 has a distal tail 122, a spacer body 150, a proximal segment 140 of a main shaft 120, and an intravascular anchor 148. In addition, a link 510 is provided between the proximal segment 140 of the main shaft 120 and the intravascular anchor 148 to connect the transcatheter device 100 to the deployment catheter (not illustrated). The intravascular anchor 148 consists of a spiral coil wound around a metal wire.

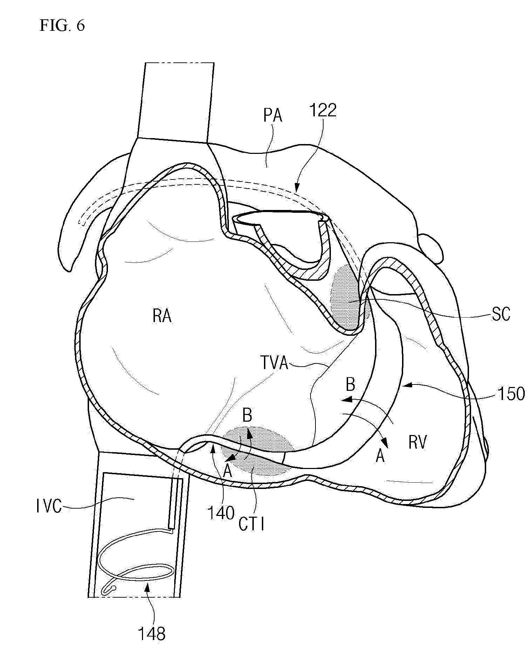

As illustrated in FIG. 6, transcatheter device 100 has a distal tail 122 anchored to a pulmonary artery (PA), an intravascular anchor 148 anchored to an inferior vena cava (IVC), and a spacer body 150 disposed across a tricuspid valve annulus (TVA) (more specifically, between leaflets of a tricuspid valve (TV)).

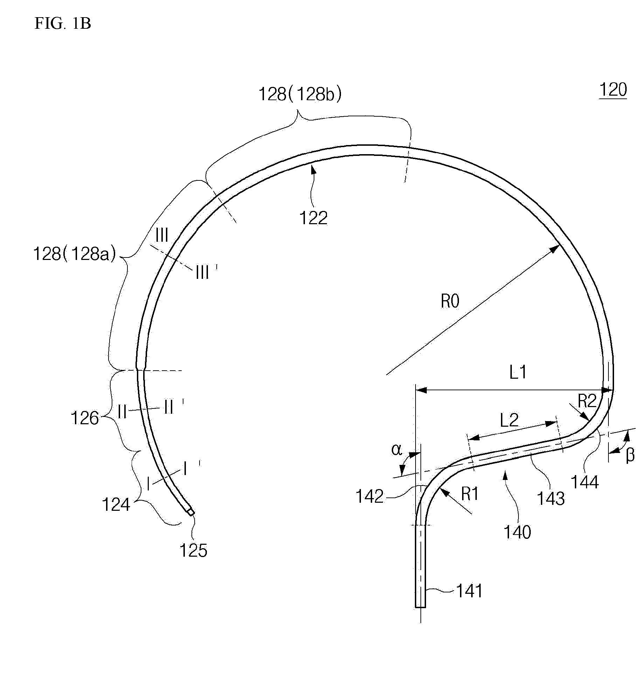

As illustrated in FIGS. 1A and 1B, the distal tail 122 has at least three segments with different flexibility. The three segments are a distal segment 124, an intermediate segment 126, and a proximal segment 128. The three segments are a distal segment 124, an intermediate segment 126, and a proximal segment 128. The distal segment 124 has a curved shape with a constant anchor curvature R0. The anchor curvature R0 is approximately 110 mm.

In addition, the distal segment 124 of the distal tail 122 is more flexible than the intermediate segment 126 and the proximal segment 128. The proximal segment 128 of the distal tail 122 is stiffer than the intermediate segment 126 and the distal segment 124. The flexibility of the distal tail 122 increases toward the distal side along a lengthwise direction of the curved shape of the distal tail 122, thereby allowing the distal tail 122 to be readily bent according to the configuration of the pulmonary artery (PA) when the distal tail 122 is inserted into the pulmonary artery (PA), which causes less trauma to the pulmonary artery (PA).

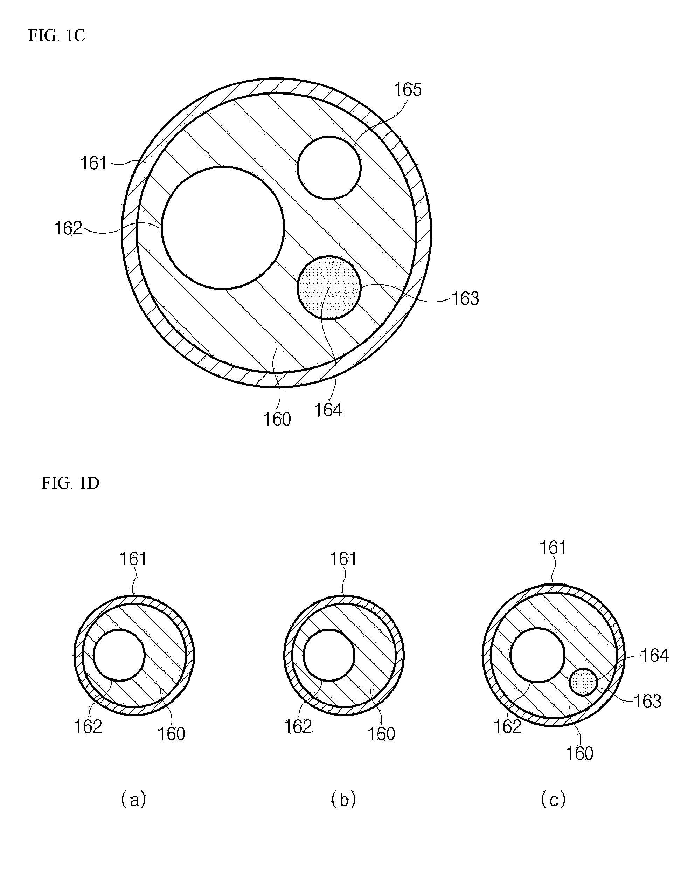

Specifically, with reference to FIG. 1D, the intermediate segment 126 and distal segment 124 of the distal tail 122 have a jacket 160, in which a first lumen 162 is formed inside the jacket 160 to guide a guidewire. In addition, a nitinol core wire 164 is not present within the jacket 160 of the intermediate segment 126 and the jacket 160 of the distal segment 124 at the distal tail 122. The jacket 160 of the intermediate segment 126 at the distal tail 122 is made of pellethane 55D (grade 55 on the Shore D hardness scale), and the jacket 160 of the distal segment 124 at the distal tail 122 is made of pellethane 90A (grade 90 on the Shore A hardness scale). A pellethane 55D material is harder than a pellethane 90A material. Diameters of intermediate segment 126 and distal segment 124 of distal tail 122 are smaller than a diameter of proximal segment 128 of distal tail 122. The jacket 160 of the intermediate segment 126 and the jacket 160 of the distal segment 124 at the distal tail 122 are surrounded by an e-PTFE layer 161. Pellethane is a brand name for a thermoplastic polyurethane elastomer that is often used in medical devices.

The proximal segment 128 of the distal tail 122 consists of a first proximal segment 128a and a second proximal segment 128b. Both the first proximal segment 128a and the second proximal segment 128a have the jacket 160 made of pellethane, and an e-PTFE layer 161 surrounding the jacket 160. Inside the jacket 160, the first lumen 162 is formed to guide the guidewire, and the second lumen 163 is formed into which the nitinol core wire 164 is inserted. The jacket 160 of the first proximal segment 128a is made of pellethane 55D (grade 55 on the Shore D hardness scale), and the jacket 160 of the second proximal segment 128b is made of pellethane 75D (grade 75 on the Shore D hardness scale). The pellethane 75D is harder than the pellethane 55D. The diameter of the proximal segment 128 is approximately 3 mm.

As described above, the distal tail 122 has a configuration in which the flexibility increases toward the distal side along the lengthwise direction of the curved shape, i.e., the second proximal segment 128b (nitinol wire + pellethane 75D jacket) > the first proximal segment 128a (nitinol wire + pellethane 55D jacket) > the intermediate segment 126 (pellethane 55D jacket) > the distal segment 124 (pellethane 90A jacket).

A total length of the distal tail 122 is approximately 15 cm. A length of the distal segment 124 is approximately 2.5 cm. A length of the intermediate segment 126 is approximately 2.5 cm. Lengths of the first proximal segment 128a and the second proximal segment 128b in the proximal segment 128 are approximately 5 cm, respectively.

The main shaft 120 of the transcatheter device 100 refers to a configuration in which the nitinol wire 164 is inserted to support the transcatheter device 100, and is sometimes described as having a proximal portion, an intermediate portion, and a distal portion for convenience of description.

The main shaft 120 corresponds to a portion from the proximal segment 140 to the proximal segment 128 of the distal tail 122 in FIGS. 1A and 1B. The proximal portion of the main shaft 120 may be a portion corresponding to the proximal segment 140, the intermediate portion of the main shaft 120 may be a portion between the proximal portion and the distal portion to which the spacer body 150 is mounted, and the distal portion of the main shaft 120 may be a portion corresponding to the proximal segment 128 of the distal tail 122. In addition, the intravascular anchor 148 is connected to the proximal portion of the main shaft 120.

The first lumen 162 for insertion of the guidewire, the second lumen 163 for insertion of the nitinol core wire 164, and a third lumen 165 for insertion of an injection tube 132 are formed to extend along the lengthwise direction inside the main shaft 120. However, the third lumen 165 for insertion of the injection tube 132 is formed only from the proximal segment 140 to a point where an injection valve 129, described below, is positioned.

The proximal segment 140 of the main shaft 120 is connected to an upper end of the intravascular anchor 148 at a point biased toward a right ventricle (RA) side rather than a center of the inferior vena cava (IVC), as illustrated in FIG. 6. In addition, the proximal segment 140 of the main shaft 120 consists of an insertion portion 141, a first bent portion 142, a connection portion 143, and a second bent portion 144, as illustrated in FIGS. 1A and 1B.

Specifically, the insertion portion 141 has a straight shape that is inserted perpendicular to the inferior vena cava (IVC), and a lower end thereof is connected to the intravascular anchor 148. The insertion portion 141 may be disposed in contact with an inner surface of the right ventricle (RA) side of the inferior vena cava (IVC). The first bent portion 142 extends to be bent with a first curvature R1 from an upper end of the insertion portion 141 toward the tricuspid valve (TV) side. The second bent portion 144 extends to be bent with a second curvature R2 from the intermediate portion of the main shaft 120 toward the inferior vena cava (IVC) side. The connection portion 143 connects the first bent portion 142 to the second bent portion 144 and extends along a cavotricuspid isthmus (CTI) between the inferior vena cava (IVC) and the tricuspid valve annulus (TVA).

The connection portion 143 has a shape that mimics the configuration of the cavotricuspid isthmus (CTI) between the inferior vena cava (IVC) and the tricuspid valve annulus (TVA), as illustrated in FIG. 6. Specifically, as illustrated in FIGS. 1A and 1B, the connection portion 141 may have an upward slope from the first bent portion 142 toward the second bent portion 144. The transcatheter device 100, having the connection portion 143 with an upward slope, may be preferably applied to the heart where the cavotricuspid isthmus (CTI) has an upward slope due to a lower position of the inferior vena cava (IVC) and a higher position of the tricuspid valve annulus (TVA). In addition, an inner angle α of the first bent portion 142 has a range of 80° to 120°. In addition, an inner angle β of the second bent portion 144 has a range of 80° to 120°.

The main shaft 120 having this proximal segment 140 is elastically deformable about the second bent portion 144 and also elastically deformable centered on the first bent portion 142 of the proximal segment 140.

Meanwhile, a position of the mounted transcatheter device 100 may be changed by a movement during the relaxation and contraction of the heart. In particular, when the right ventricle (RV) contracts, the intermediate and distal portions are experienced a movement that is pushed toward the right ventricle (RV) side. This may cause the intermediate and distal portions to deform in a direction of arrow A in FIG. 6, centered on the second bent portion 144, and deform in the direction of arrow A in FIG. 6, centered on the first bent portion 142.

As described above, since the intermediate portion and the distal portion of the transcatheter device 100 are capable of elastic deformation centered on the second bent portion 144 of the proximal segment 41 and are also capable of elastic deformation centered on the first bent portion 142 of the proximal segment 41, even in this case, a restoring force is generated that pushes the intermediate portion and the distal portion in a direction of arrow B, which is opposite to the direction of arrow A above. As a result, the distal tail 122, including the distal portion, may be closely in contact inside the pulmonary artery (PA), and further, a point where the distal portion and intermediate portion meet may be closely in contact with a supraventricular crest (SUV) (see FIG. 6). As a result, the transcatheter device 100 and spacer 120 may be stably retained in a desired position with respect to the movement of the heart, particularly during contraction of the heart.

In addition, the first curvature R1 of the first bent portion 142 and the second curvature R2 of the second bent portion 144 are each determined by the configuration of the heart in the corresponding portion, and may be the same or different. However, when the first curvature R1 of the first bent portion 142 and the second curvature R2 of the second bent portion 144 are the same, it is easier to be manufactured.

Meanwhile, a horizontal length L1 of the proximal segment 140 of the main shaft 120, that is, a length L1 between the first bent portion 142 and the second bent portion 144, preferably has a range that covers the cavotricuspid isthmus (CTI). In this embodiment, the horizontal length L1 of the proximal segment 41 has a range of 30 to 70 mm. In addition, an extension length L2 in the lengthwise direction of the connection portion 143, which is positioned between the first bent portion 142 and the second bent portion 144, has a range of 30 to 50 mm.

FIG. 1C is a cross-sectional view taken along E-E' in FIG. 1A, illustrating a cross-sectional view of the main shaft of the transcatheter device. As illustrated in FIG. 1C, the proximal segment 140 of the main shaft 120 has the jacket 160 made of pellethane 90A (grade 90 on the Shore A hardness scale) and the e-PTFE layer 161 surrounding the jacket 160, in which the first lumen 162 is formed inside the jacket 160 to guide the guidewire. The diameter D1 of the proximal segment 140 of the main shaft 120 is approximately 3 mm.

In addition, the proximal segment 140 of the main shaft 120 has the second lumen 163 formed therein, into which the nitinol core wire 164 is inserted to structurally support the main shaft 120 over an entire length of the main shaft 120. This nitinol core wire 164 extends to the proximal segment 128 of the distal tail 122 and imparts a preformed curved shape to the distal tail 122. In addition, the third lumen 165 is formed in the main shaft 120 into which the injection tube 132, described below, is inserted.

In addition, as can be seen from FIG. 1C and (c) of FIG. 1D, the proximal segment 140 of the main shaft 120 has a similar structure and similar material composition to the proximal segment 128 of the distal tail 122, except that the lumen of the injection tube 165 is formed. Therefore, the proximal segment 128 of the distal tail 122 and the proximal segment 140 of the main shaft 120 have similar strengths.

The transcatheter device 100 has a series of radiopaque markers. The proximal segment 140 of the main shaft has a radiopaque strip (not illustrated). The distal tail 122 has a series of radiopaque bands. In addition, a tip of the distal tail 122 is provided with a radiopaque band.

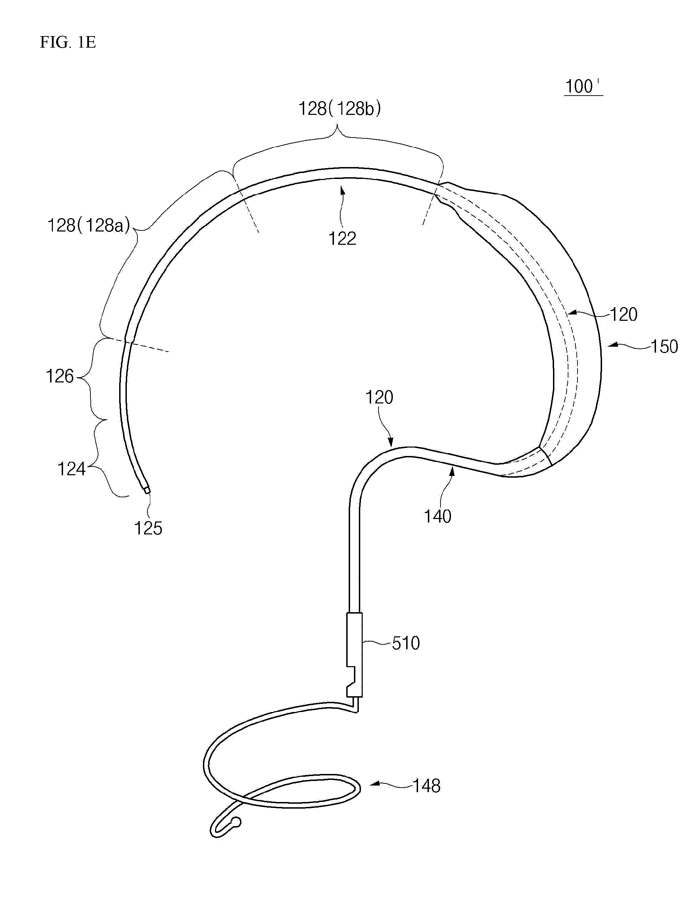

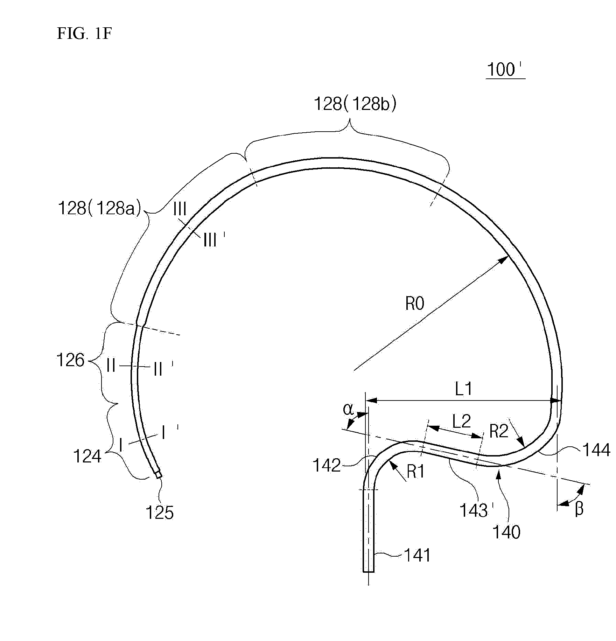

FIG. 1E is a view illustrating another example of the transcatheter device for treating tricuspid valve regurgitation, and FIG. 1F is a view illustrating the main shaft and distal tail of the transcatheter device in FIG. 1E. A transcatheter device 100' illustrated in FIGS. 1E and 1F differs from the transcatheter device 100 illustrated in FIGS. 1A to 1D only in the configuration of the connection portion 143' consisting of the proximal segment 140 of the main shaft 120, which is otherwise identical.

Specifically, the connection portion 143 of the transcatheter device 100 illustrated in FIGS. 1A to 1D differs in that it has an upward slope from the first bent portion 142 toward the second bent portion 144, while the connection portion 143' of the transcatheter device 100' illustrated in FIGS. 1E and 1F has a downward slope from the first bent portion 142 toward the second bent portion 144. The transcatheter device 100, having the connection portion 143' with a downward slope, may be preferably applied to the heart where the cavotricuspid isthmus (CTI) has a downward slope due to a higher position of the inferior vena cava (IVC) and a lower position of the tricuspid valve annulus (TVA). In addition, the inner angle α of the first bent portion 142 has a range of 80° to 120°, and the inner angle β of the second bent portion 144 has a range of 80° to 120°. Further, the extension length L2 in the lengthwise direction of the connection portion 143', which is positioned between the first bent portion 142 and the second bent portion 144, has a range of 30 to 50 mm.

Meanwhile, all of the main shaft 120, distal tail 122, and intravascular anchor 148 are surrounded by the e-PTFE layer 161 of a constant thickness, which is made of e-PTFE. As illustrated in FIGS. 1A, 1B, 1E, and 1F, the e-PTFE layer 161 extends from the tip of the distal tail 122 by a certain distance to form a round wrapped tip 125. That is, since the tip 124 is more protruding than the tip of the distal segment 124 of the distal tail 122, consists of only the e-PTFE layer, and is wrapped round, when inserted into the pulmonary artery (PA), the tip 125 will not traumatize the pulmonary artery (PA) even touching the pulmonary artery (PA).

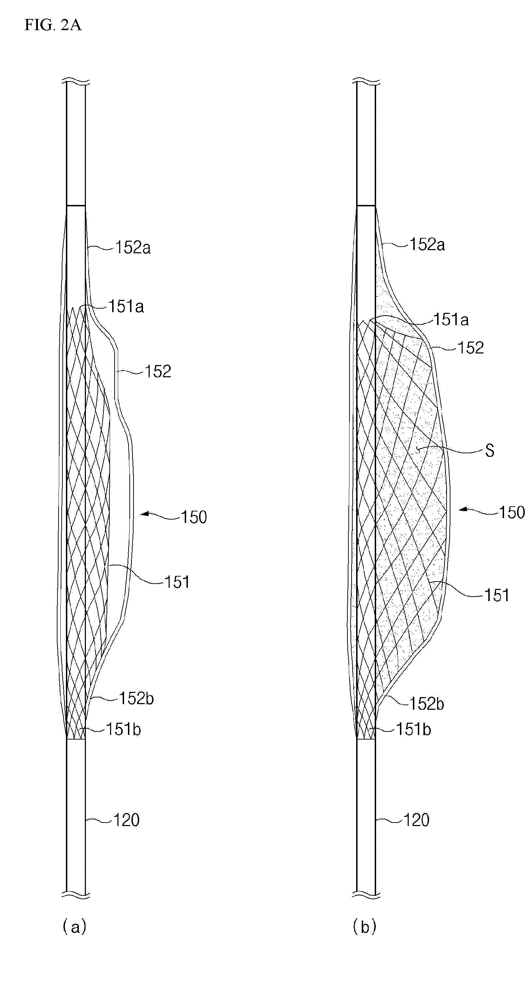

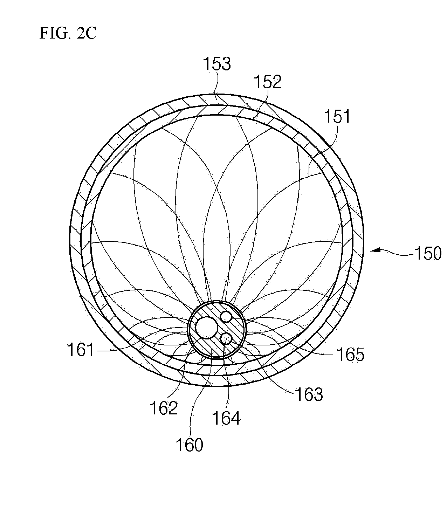

FIGS. 2A and 2C are views illustrating an example of the spacer body 150 in detail. In addition, (a) and (b) of FIG. 2A are views illustrating a state in which the spacer body 150 is not filled with a saline solution and a state in which it is filled with a saline solution, respectively. The spacer body 150 has a distal end 151a and a proximal end 151b, and a mesh structured body 151 formed of a wire. The main shaft 120 passes through the spacer body 150. One side of the distal end 151a and the proximal end 151b of the main shaft 120 is fixedly mounted to the main shaft 120 and the other side is not fixedly mounted to the main shaft 120 and is movable with respect to the main shaft 120 in an axial direction. In this embodiment, the proximal end 151b of the spacer body 150 is fixed and the distal end 151b is movable.

The mesh structured body 151 is made of a shape memory alloy or the like, and maintains an expanded predetermined configuration in the absence of an external force. In addition, the mesh structured body 151 may contract when an external force is applied, in which case the other side that is not fixed to the main shaft 120 may move along the main shaft 120. The mesh structured body 151 may contract or expand during a deployment process.

As illustrated in FIG. 2A, the spacer body 150 has a balloon 152 having a distal end 152a and a proximal end 152b. Both ends of the distal end 152a and the proximal end 152b of the balloon 152 are fixedly mounted to the main shaft 120. The balloon 152 is formed as an enclosed structure that covers the mesh structured body 151. An enclosed internal space of the balloon 152 may be filled with a saline solution S.

As illustrated in (a) of FIG. 2A, even when the internal space of the balloon 152 is not filled with the saline solution S, the balloon 152 may retain the shape thereof to some extent by the mesh structured body 151 positioned inside the balloon 152. In addition, as illustrated in (b) of FIG. 2A, when the internal space of the balloon 152 is filled with the saline solution S, the balloon 152 expands, and the mesh structured body 151 expands accordingly.

The balloon 152 is formed to adjust a shape thereof in response to the patient's heart (specifically, a size of a regurgitation space in which tricuspid valve regurgitation occurs). In this embodiment, the balloon 152 has a shape in which the right ventricle (RV) side is larger than the right atrium (RA) side with respect to the main shaft 120 in the expanded state.

A volume of the spacer body 150 may be adjusted depending on an amount of saline solution S injected into the balloon 152. Therefore, a size of the spacer body 150 may be adjusted to fit the size of the regurgitation space in which the tricuspid valve regurgitation occurs, or the like. As a result, the operator may simply adjust the amount of saline solution S injected without the need of retrieval when the spacer body 150 does not fit the size of the regurgitation space. In addition, air rather than the saline solution S may be filled inside the balloon 152.

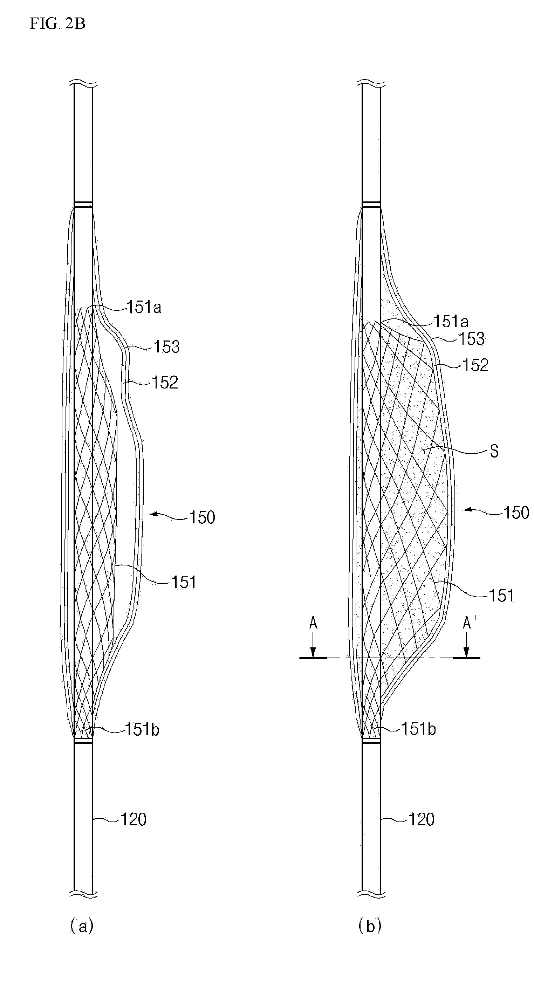

FIG. 2B is a view illustrating another example of the spacer body 150, in which (a) and (b) of FIG. 2B illustrate a state in which the spacer body 150 is not filled with the saline solution and a state in which the spacer body 150 is filled with the saline solution, respectively. The spacer body 150 illustrated in FIG. 2B further includes an e-PTFE layer 153 formed to cover the balloon 152 with respect to the spacer body 150 illustrated in FIG. 2A. The e-PTFE layer 153 is formed to expand with the balloon 152 as the balloon 152 is inflated by the saline solution being supplied. The e-PTFE layer 153, like the balloon 152, is formed to adjust the shape of the balloon 152 in response to the patient's heart (specifically, the size of the regurgitation space where the tricuspid valve regurgitation occurs).

FIG. 2C is a cross sectional view taken along A-A' of the spacer body 150 in (b) of FIG. 2B. When the spacer body 150 expands within the tricuspid valve, the balloon 152 and e-PTFE layer 153 are formed to cover the expanded mesh structured body 151.

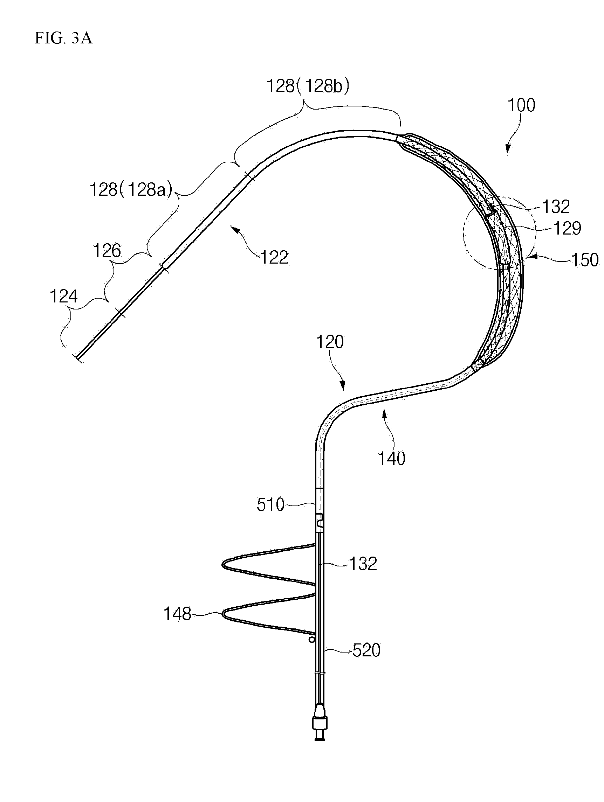

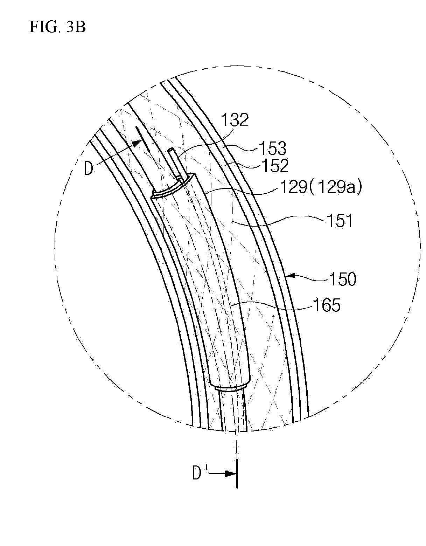

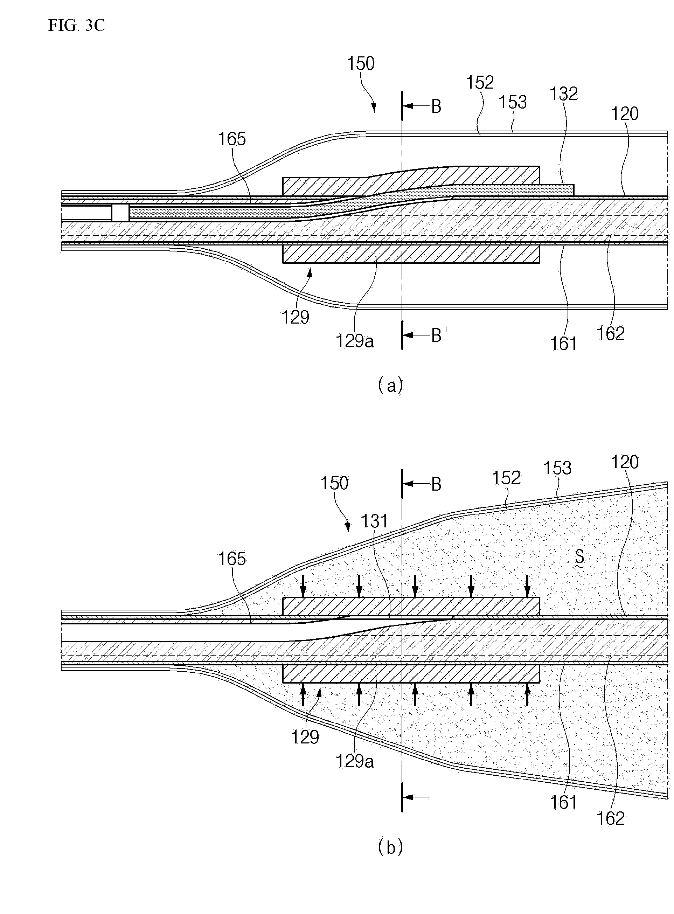

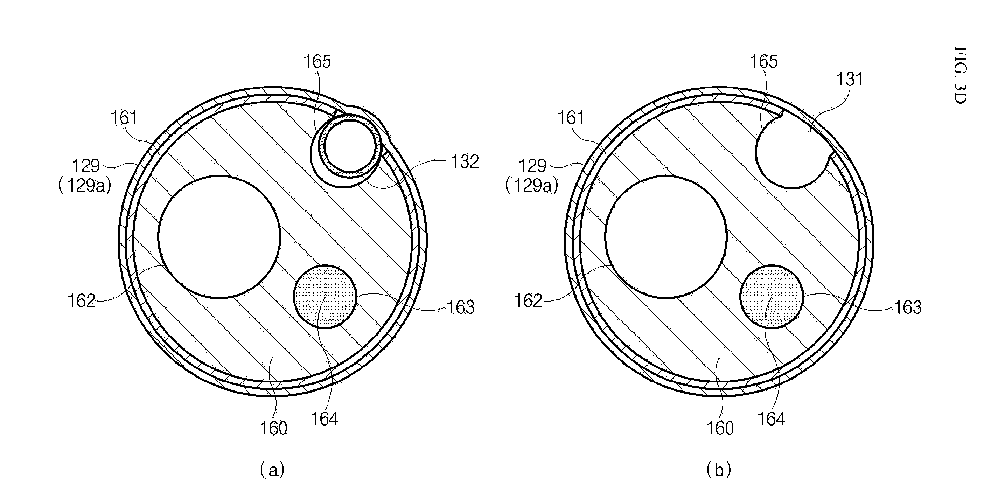

FIGS. 3A and 3B are views illustrating the injection valve 129 and the injection tube 132, in which FIG. 3B is an enlarged view of the injection valve 129 in FIG. 3A, FIG. 3C is a cross-sectional view of the injection valve 129 taken along D-D' in FIG. 3B, and FIG. 3d is a cross-sectional view of the injection valve 129 taken along B-B' in FIG. 3C. In addition, (a) in FIG. 3C and (a) in FIG. 3D illustrate a state in which the injection tube 132 is inserted and the injection valve 129 is open, and (b) in FIG. 3C and (b) in FIG. 3D illustrate a state in which the injection tube 132b is removed and the injection valve 129 is enclosed.

The injection valve 129 is formed of a silicon band 129a with elasticity. The silicon band 129a surrounds an outer side of the main shaft 120. The injection tube 132 may be inserted between the silicon band 129a and the main shaft 120.

An injection hole 131 is formed in the main shaft 120 that communicates with the third lumen 165 and the internal space of the spacer body 150. The silicon band 129a covers the injection hole 131. The injection tube 132 may pass through the injection hole 131.

The third lumen 165 extends from the proximal segment 140 of the main shaft 120 to the injection hole 131, and the injection tube 132 advances along the third lumen 165 and passes through the injection hole 131 to be exposed to the internal space of the spacer body 150 (see FIG. 3B and (a) of FIG. 3C).

With a tip of the injection tube 132 exposed to the internal space of the spacer body 150, the silicon band 129a extends in a diameter direction due to the injection tube 132, which causes the injection valve 129 to be in an open state (see (a) of FIG. 3C and (a) of FIG. 3D). When the saline solution S is injected into the internal space of the spacer body 150 through the injection tube 132 in this open state, the balloon 152 is inflated. As the balloon 152 expands, the e-PTFE layer 153 also expands.

When the injection of the saline solution S through the injection tube 132 is completed, and the injection tube 132 is withdrawn to the outside and retrieved, the silicon band 129a contracts and is closely in contact with an outer surface of the main shaft 120. As a result, the injection hole 131 is blocked by the silicon band 129a, resulting in the third lumen 165 being closed with the injection valve 129 being closed (see (d) in FIG. 3C and (d) in FIG. 3D).

In the closed state of the injection valve 129, the saline solution S is filled inside the spacer body 150, which has a predetermined pressure, so that the silicon band 129a can be more firmly and closely in contact with the outer surface of the main shaft 120 due to the pressure. This more securely prevents the saline solution S filled inside the spacer body 150 from escaping through the third lumen 165.

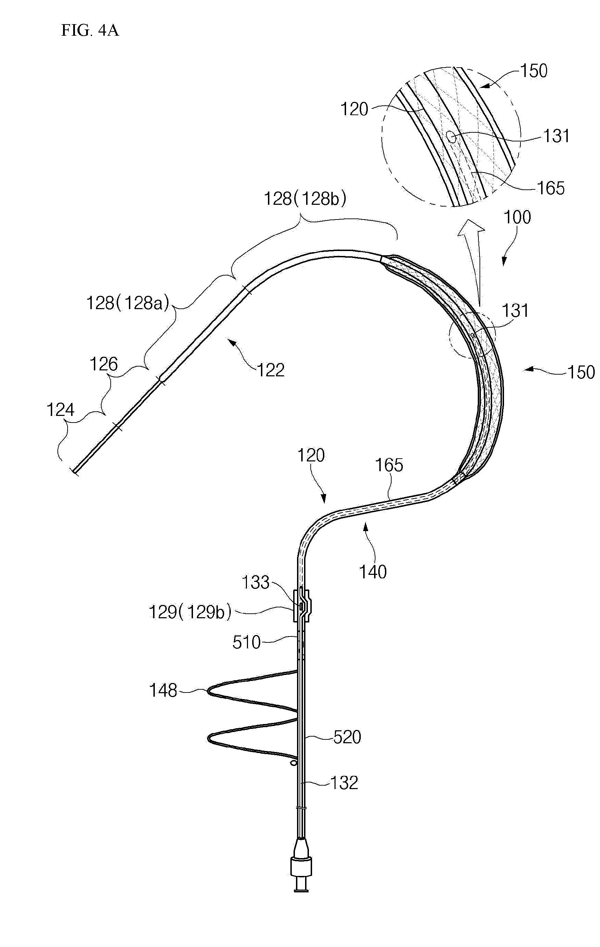

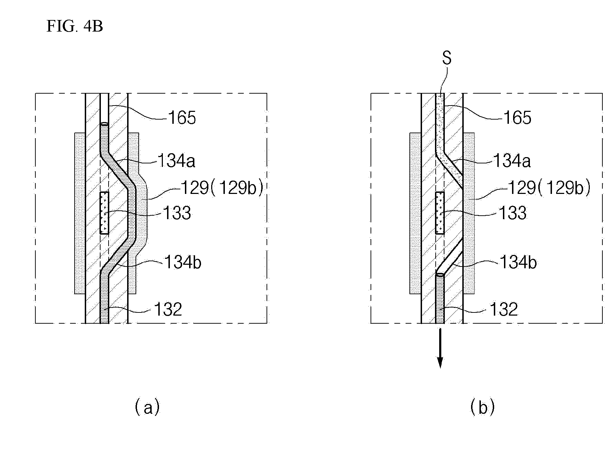

Next, FIG. 4A is a view illustrating an embodiment of the transcatheter device in which the injection valve is mounted on the proximal portion of the main shaft, and FIG. 4B is an enlarged view of a portion in which the injection valve in FIG. 4A is mounted, illustrating an open state ((a) of FIG. 4B) and a closed state ((b) of FIG. 4B) of the injection valve.

With reference to FIGS. 4A and 4B, the injection valve 129 is formed of the silicon band 129b with elasticity and wraps around a point on the proximal portion of the main shaft 120. The injection hole 131 is formed in the main shaft 120 that communicates with the third lumen 165 and the internal space of the spacer body 150. That is, the third lumen 165 of the main shaft 120 extends from the proximal portion of the main shaft 120 to the injection hole 131.

In addition, the proximal portion of the main shaft 120 is provided with a closure portion 133 that closes off a section of the third lumen 165. At both ends of the closure portion 133 are formed communication holes 134a and 134b that communicate with the third lumen 165 and the outside of the main shaft 120, and through which the injection tube 132 passes.

The silicon band 129b consisting of the injection valve 129 has a size in the lengthwise direction covering the closure portion 133 and the communication holes 134a and 134b, and is capable of closing the communication holes 134a and 134b upon contraction.

As illustrated in (a) of FIG. 4B, the injection tube 132 is inserted through the third lumen 165 and exits the outer side of the main shaft 120 through the communication hole 134a just before the closure portion 133, and is inserted again through the communication hole 134b into the third lumen 165 on the inner side of the main shaft 120. In this state, the silicon band 129b extends in the diameter direction (the open state of the injection valve 129).

In the open state of the injection valve 129, the injection tube 132 is in communication with the injection hole 131 positioned within the spacer body 150 through the third lumen 165. Therefore, when the saline solution S is injected through the injection tube 132, the saline solution S passes through the third lumen 165 and enters the space within the spacer body 150 through the injection hole 131 to expand the balloon 152. As the balloon 152 expands, the e-PTFE layer 153 also expands.

When the injection of the saline solution S into the spacer body 150 is completed, the injection tube 132 is withdrawn to the outside and retrieved. When the injection tube 132 is withdrawn to the outside and retrieved, the injection tube 132 is withdrawn from within the silicon band 129a, causing the silicon band 129a to contract, as illustrated in (b) of FIG. 4B. This causes the silicon band 129a to be closely in contact with the outer surface of the main shaft 120, which closes the communication holes 134a and 134b (in the closed state of the injection valve 129), and prevents the injected saline solution S from escaping to the outside.

As described above, when the injection valve 129 is provided in the proximal portion of the main shaft 120, it has an advantage in that it is not necessary to insert the injection tube 132 up to the injection hole 131 inside the spacer body 150. In addition, the silicon band 129b need not necessarily be provided on the outer side of the main shaft 120, and may be provided on an outer side of an upper link 510a of a link 510 described below.

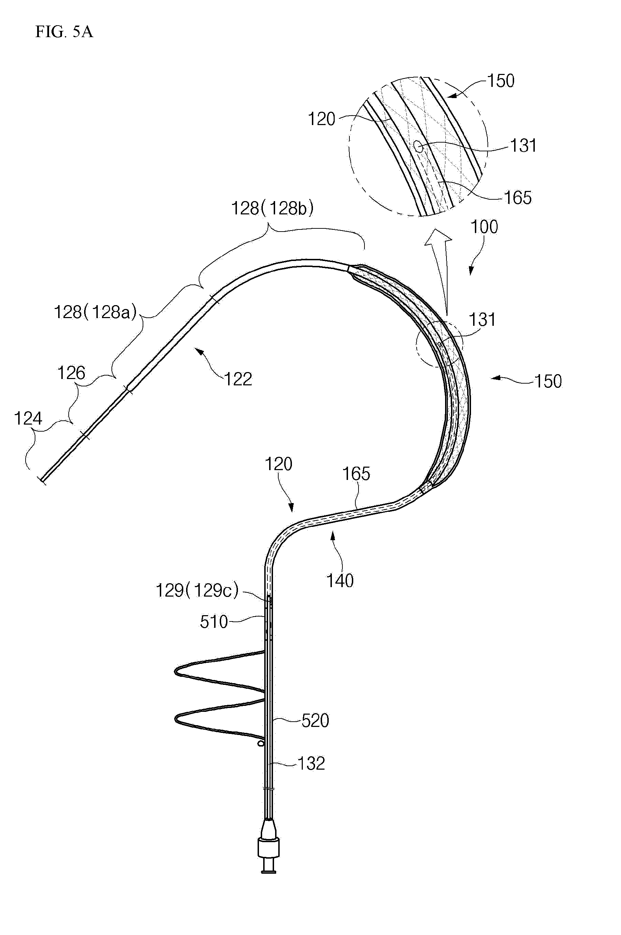

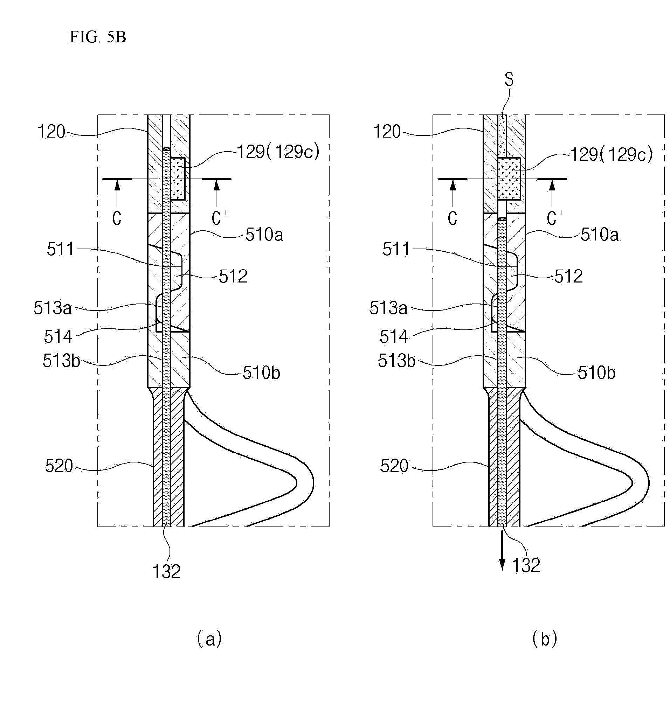

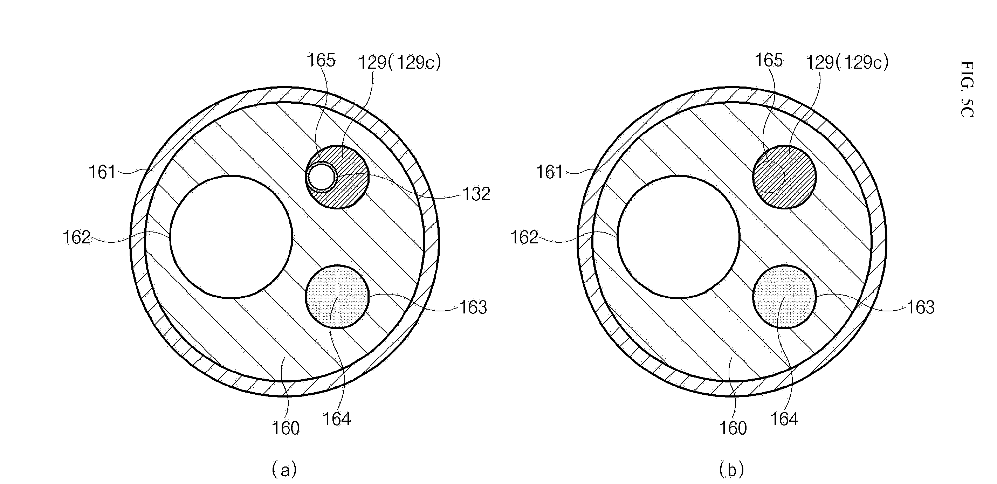

In addition, FIGS. 5A, 5B, and 5C are another embodiment of the transcatheter device 100 using the injection valve 129 provided within the proximal portion of the main shaft 120. FIG. 5B is an enlarged view of the injection valve 129 in FIG. 5A, and FIG. 5C is a cross-sectional view taken along C-C' in FIG. 5B. In addition, (a) of FIG. 5B and (a) of FIG. 5C illustrate the open state of the injection valve 129, and (b) of FIG. 5B and (b) of FIG. 5C illustrate the closed state of the injection valve 129.

The injection valve 129 is formed of a silicon body 129c in a cylindrical shape with elasticity. The silicon body 129c is provided on the inner side of the main shaft 120 around the proximal portion of the main shaft 120. The silicon body 129c has a cross-sectional size that is capable of covering the third lumen 165 in the extended state. Since the silicon body 129C has elasticity, the silicon body 129c may fill the third lumen 165 in the extended state to make the third lumen 165 to be in the closed state, and make, in the contracted state, the third lumen 165 to be in the open state. In addition, the silicon body129c need not necessarily be provided on the inner side of the main shaft 120, and may be provided on an inner side of an upper link 510a of a link 510 described below.

As illustrated in FIG. 5A, the injection hole 131 is formed in the main shaft 120 that communicates with the third lumen 165 and the internal space of the spacer body 150. That is, the third lumen 165 of the main shaft 120 extends from the proximal portion of the main shaft 120 to the injection hole 131.

As illustrated in (a) of FIG. 5B and (a) of FIG. 5C, when the injection tube 132 is inserted through the third lumen 165, the silicon body 129C contracts, which results in the open state in which the injection tube 132 may pass through (the open state of the injection valve 129). When the saline solution S is injected through the injection tube 132 in this open state, the saline solution S is injected through the third lumen 165 and the injection hole 131 into the internal space of the spacer body 150 to expand the balloon 152.

When the injection is completed and the injection tube 132 is withdrawn as illustrated in (b) of FIG. 5B and (b) of FIG. 5C, the silicon body 129c expands, which causes the closed state in which the third lumen 165 is blocked (the closed state of the injection valve 129). Since the saline solution S within the balloon 152 does not escape in this closed state, the balloon 152 remains in the expanded state with a predetermined volume by the filled saline solution S.

Meanwhile, with reference to FIGS. 5A and 5B, a link 510 is illustrated with an upper link 510a provided on the transcatheter device 100 and a lower link 510b provided on an upper end of a pusher 520 of a deployment catheter (not illustrated) that catches on each other. Passages 513a and 513b are formed in the upper link 510a and lower link 510b into which the injection tube 132 is inserted along a lengthwise direction. When the upper link 510a and lower link 510b are linked, the passages 513a and 513b are connected, and these passages 513a and 513b are in communication with the third lumen 165.

The injection tube 132 passes through the lower link 510b and upper link 510a and is inserted into the third lumen 165 of the transcatheter device 100. The state in which the upper link 510a and the lower link 510b are linked is maintained by the injection tube 132 passing through the upper link 510a and the lower link 510b, which results in the connection of the transcatheter device 100 and the pusher 520 of the deployment catheter. With the transcatheter device 100 and the pusher 520 of the deployment catheter connected, the operator may push the transcatheter device 100 through the pusher 520 to advance the transcatheter device 100 to a desired position.

In addition, when the injection tube 132 is withdrawn from the upper link 510a and lower link 510b, the linked state of the upper link 510a and lower link 510b is released, thereby releasing the connection between the transcatheter device 100 and the pusher 520 of the deployment catheter. The operator may deploy the transcatheter device 100 in the desired position, and after the saline solution is injected through the injection tube 132, the operator may release the link between the upper link 510a and the lower link 510b by withdrawing the injection tube 132, and then withdraw the pusher 520 of the deployment catheter and the lower link 510b, which are separated from the upper link 510a.

The upper link 510a has a concave portion 511 formed thereon, and the lower link 510b has a convex portion 512 formed thereon that catches on the concave portion 511. The upper link 510a and lower link 510b are linked by the concave portion 511 of the upper link 510a being engaged and caught by the convex portion 512 of the lower link 510b. In case of a structure in which the upper link 510a and the lower link 510b catch each other, the upper link 510a may have a convex portion formed thereon and the lower link 510b may have a concave portion formed thereon, or other configurations may be used.

Meanwhile, each surface of the concave portion 511 and the convex portion 512 that is in contact with each other when the upper link 510a and the lower link 510b are linked is a sloped surface that is inclined with respect to the lengthwise direction. Therefore, catching and separating of the upper link 510a and lower link 510b is made easier.

In addition, a lower end of the upper link 510a has an edge 514 formed as a curved surface. When the retrieval occurs in the state in which the lower link 510b is separated, the lower end of the upper link 510a hits the blood vessel, which does not cause trauma to the blood vessel because the edge 514 has a curved surface.

In addition, when the transcatheter device 100 is withdrawn from the body and retrieved, the saline solution S in the spacer body 150 needs to be withdrawn from the spacer body 150 to contract the spacer body 150. In this case, a needle with a pointed tip wrapped in a delivery sheath can be approached to the outer side of the spacer body 150, and then the needle can be exposed from the delivery sheath to make a hole in the balloon 152 and e-PTFE layer 153 of the spacer body 150 and allow the saline solution S to drain from the spacer body 50 through the hole.

The description and examples described above are intended to illustrate the present invention only and are not intended to limit the scope of the claims. Each of the disclosed aspects and embodiments of the present invention may be considered individually or in combination with other aspects, embodiments, and modifications of the present invention. Further, unless otherwise specified, the steps of the methods of the present invention are not limited to any specific sequence of execution. Modifications to the disclosed embodiments that include the concepts and essence of the present invention may be accomplished by those skilled in the art, and such modifications are included within the scope of the present invention.

In this specification, any use of the word "or" is intended to be inclusive and is equivalent to the expression "and/or" unless the context clearly indicates otherwise. Accordingly, for example, the expression 'A or B' means either A, or B, or both A and B. Similarly, for example, the expression "A, B, or C" means A, or B, or C, or a combination of A, B, and C.

**<Description of Reference Numerals>**

| | | | |
|---|---|---|---|
| 100, 100' : | Transcatheter device | 120 : | Main shaft |
| 122 : | Distal tail | 124 : | Distal segment |
| 126 : | Intermediate segment | 128 : | Proximal segment |
| 129 : | Injection valve | 129a and 129b : | silicon band |
| 129c : | Silicon body | 131 : | Injection hole |
| 132 : | Injection tube | 140 : | Proximal segment of main shaft |
| 141 : | Insertion portion | 142 : | First bent portion |
| 143, 143' : | Connection portion | 144 : | Second bent portion |
| 148 : | Intravascular anchor | 150 : | Spacer body |
| 151 : | Mesh structured body | 152 : | Balloon |
| 153 : | e-PTFE layer | 160 : | Jacket |
| 161 : | e-PTFE layer | 162 : | First lumen |
| 163 : | Second lumen | 164 : | Nitinol core wire |
| 165 : | Third lumen | 510 : | Link |
| 510a : | Upper link | 510b: | lower link |
| R1 : | First curvature | R2 : | second curvature |
| IVC : | Inferior vena cava | RV : | Right ventricle |
| RA : | Right atrium | PA: | Pulmonary artery |
| CTI : | Cavotricuspid isthmus | TV : | Tricuspid valve |
| TVA : | Tricuspid valve annulus | SC : | Supraventricular crest |

## Claims

1. A transcatheter device comprising:
a main shaft comprising a proximal portion, an intermediate portion, and a distal portion, and having a first lumen for insertion of a guidewire, a second lumen for insertion of a nitinol wire, and a third lumen for insertion of an injection tube formed therein;
a distal tail comprising a distal portion of the main shaft;
a spacer body mounted on the intermediate portion positioned between the distal portion and the proximal portion of the main shaft,
wherein the proximal portion of the main shaft comprises a proximal segment and an intravascular anchor connected to the proximal segment, and
wherein the spacer body comprises:
a mesh structured body configured contractible or expandable and to be expanded to have a predetermined configuration in the absence of an external force from an environment; and
a balloon configured to cover the mesh structured body to maintain an enclosed state, and to be controlled in size depending on an amount of a saline solution injected into an internal space thereof.

2. The transcatheter device of claim 1, wherein the spacer body further comprises an e-PTFE layer the configured to cover the balloon.

3. The transcatheter device of claim 1, wherein the main shaft further comprises an injection hole configured to communicate the third lumen with an internal space of the spacer body, and an injection valve configured to open and close the communication between the third lumen and the injection hole, by the injection tube being inserted or withdrawn.

4. The transcatheter device of claim 3, wherein the injection valve comprises a silicon band provided on an outer side of the main shaft inside the spacer body.

5. The transcatheter device of claim 3, wherein the injection valve comprises a silicon band provided on an outer side of the main shaft in the proximal portion of the main shaft.

6. The transcatheter device of claim 3, wherein the injection valve comprises a silicon body provided on an inner side of the main shaft in the proximal portion of the main shaft.

7. The transcatheter device of any one of claims 1 to 6, wherein in the proximal portion of the main shaft, the proximal segment comprises:
an insertion portion inserted into an inferior vena cava (IVC) and connected to the intravascular anchor;
a first bent portion extending to be bent with first curvature R1 from an upper end of the insertion portion toward a tricuspid valve (TV) side;
a second bent portion extending to be bent with second curvature R2 from the intermediate portion of the main shaft toward an inferior vena cava (IVC) side; and
a connection portion configured to connect the first bent portion to the second bent portion and extending along a cavotricuspid isthmus (CTI) between the inferior vena cava (IVC) and the tricuspid valve (TV).

8. The transcatheter device of claim 7, wherein a horizontal length L1 of the proximal segment in the proximal portion of the main shaft has a range that covers the cavotricuspid isthmus (CTI) between the inferior vena cava (IVC) and the tricuspid valve (TV).

9. The transcatheter device of claim 8, wherein the horizontal length L1 of the proximal segment has a range of 30 to 70 mm.

10. The transcatheter device of claim 8, wherein an extension length L2 of the connection portion of the proximal segment has a range of 30 to 50 mm.

11. The transcatheter device of claim 7, wherein an inner angle α of the first bent portion has a range of 80° to 120°.

12. The transcatheter device of claim 7, wherein an inner angle α of the second bent portion has a range of 80° to 120°.

13. A coaptation assembly comprising:
the transcatheter device of claim 1;
the guidewire configured to move through the first lumen of the main shaft;
a delivery sheath movable to cover the spacer body and the intravascular anchor; and
a deployment catheter configured for intravascular deployment of the transcatheter device.

14. The coaptation assembly of claim 13, comprising:
an upper link provided on the proximal portion of the main shaft in the transcatheter device; and
a lower link connected to a pusher of the deployment catheter and capable of being caught on or separated from the upper link,
wherein a passage through which the injection tube passes is communicated to the upper link and the lower link that are in a linked state.

15. A method of treating tricuspid valve regurgitation in a patient's heart using the transcatheter device of claim 1, the method comprising:
inserting the transcatheter device into a femoral vein;
advancing the transcatheter device through an inferior vena cava;
advancing the transcatheter device through a right atrium of the heart;
advancing the transcatheter device across a tricuspid valve into a right ventricle of the heart;
advancing the transcatheter device toward a pulmonary artery;
advancing the distal tail into the pulmonary artery by a distance of at least 10 cm;
positioning the spacer body between leaflets of the tricuspid valve;
lodging the intravascular anchor within the inferior vena cava; and
injecting a saline solution into the spacer body, and adjusting an amount of the injected saline solution such that the spacer body has a predetermined size and volume.

16. The method of claim 15, further comprising:
withdrawing, upon retrieval of the transcatheter device, the saline solution filled within the spacer body by accessing a needle with a pointed tip to an outer side of the spacer body to form a hole in the spacer body.
